# EUROPÄISCHE PATENTSCHRIFT

(11) **EP 1 279 704 B1**
(45) Veröffentlichungstag und Bekanntmachung des Hinweises auf die Patenterteilung: **04.08.2004**
(21) Anmeldenummer: 02012518.3
(22) Anmeldetag: 05.06.2002
(51) Int. Cl.: C09C 1/56

(54) **Verfahren zur Herstellung von modifiziertem Russ**
Method for producing modified carbon black
Procédé de production de noir de carbone modifié

(30) Priorität: 25.07.2001 DE 10136043
(43) Veröffentlichungstag der Anmeldung: 29.01.2003
(73) Patentinhaber: Degussa AG, 40474 Düsseldorf (DE)
(72) Erfinder: Karl, Alfons, Dr., 63584 Gründau (DE); McIntosh, Ralph, 63457 Hanau (DE); Kalbitz, Werner, 63517 Rodenbach (DE); Kleinhenz, Horst, 63538 Grosskrotzenburg (DE); Tauber, Gerd, 63500 Seligenstadt (DE); Lüdtke, Stephan, 63599 Biebergemünd (DE)

(56) Entgegenhaltungen:
- EP-A- 1 061 107
- EP-A- 1 072 654
- US-A1- 2001 004 871
- US-B1- 6 214 100

## Beschreibung

Die Erfindung betrifft ein Verfahren zur Herstellung von modifiziertem Ruß.

Aus EP 0569503 ist ein Verfahren zur Oberflächenmodifizierung von kohlenstoffhaltigem Material mit aromatischen Gruppen durch elektrochemische Reduktion eines Diazoniumsalzes bekannt.

Es ist bekannt, Ruß mit organischen Gruppen herzustellen, indem man die organischen Gruppen mittels einer Diazoniumgruppe, die über das primäre Amin erzeugt wird, mit dem Ruß verknüpft (WO 96/18688).

Die bekannten Verfahren haben den Nachteil, daß Verbindungen, die sowohl Hydroxi- als auch Aminogruppen enthalten, nicht mit dem Ruß reagieren.

Aufgabe der Erfindung ist es, ein Verfahren zur Herstellung von modifiziertem Ruß zur Verfügung zu stellen, wobei der Ruß auch mit Verbindungen, die mindestens eine Hydroxi und mindestens eine Aminogruppe aufweisen, reagiert.

Gegenstand der Erfindung ist ein Verfahren zur Herstellung von modifiziertem Ruß, welches dadurch gekennzeichnet ist, daß man eine Rußdispersion, enthaltend Ruß, Wasser und Netzmittel, mit einer sauren, wäßrigen Lösung oder Suspension eines primären Amins, das mindestens eine Hydroxi und mindestens eine Aminogruppe aufweist, mischt und anschließend mit Natriumnitrit-Lösung umsetzt.

Als Ruß können Furnaceruß, Gasruß, Channelruß, Flammruß, Thermalruß, Acetylenruß, Plasmaruß, Inversionsruße, bekannt aus DE 195 21 565, Si-haltige Ruße, bekannt aus WO 98/45361 oder DE 19613796, oder metallhaltige Ruße, bekannt aus WO 98/42778, Lichtbogenruß und Ruße, die Nebenprodukte chemischer Produktionsprozesse sind, verwendet werden. Der Ruß kann durch vorgelagerte Reaktionen, beispielsweise durch eine Oxidation, aktiviert werden.

Es können Farbruße eingesetzt werden.

Weitere Ruße können sein: Leitfähigkeitsruß, Ruß zur UV-Stabilisierung, Ruß als Füllstoff in anderen Systemen als Kautschuk, wie zum Beispiel in Bitumen, Kunststoff, Ruß als Reduktionsmittel, in der Metallurgie.

Als Netzmittel können anionische, kationische und/oder nichtionische Netzmittel verwendet werden.

Als anionisches Netzmittel kann Tamol verwendet werden.

Als kationisches Netzmittel kann Akypoquat 132 (kationischer Fettester (CTFA: Lauroyl PG-Trimonium Chloride)) der Firma Kao Chemicals GmbH, Bayowet FT 738 VP AC 2023 (Quarternäres Fluoralkylammoniumiodid) der Firma Bayer AG, DP2-7949 (Wässrige Lösung kationischer Homopolymere) der Firma Ciba Geigy Chemicals, DP7-7961 (Wässrige Lösung kationischer Polymere) der Firma Ciba Geigy Chemicals, DP7-7962 (Wässrige Lösung kationischer Polymere) der Firma Ciba Geigy Chemicals, DP7-7963 (Wässrige Lösung kationischer Polymere) der Firma Ciba Geigy Chemicals, Epikuron 200 (Phosphatidylcholin) der Firma Lukas Meyer, Ethoxamine SF 11 (Etoxyliertes Fettamin mit 11 Mol Ethylenoxid) der Firma Witco, Ethoxamine SF 15 (Ethoxyliertes Fettamin mit 15 Mol Ethylenoxid) der Firma Witco, Forbest 13 (Compound neutr., saurer Polyester und Fettalkohol) der Firma Lukas Meyer, Forbest 610 (Carbonsäure-Diamin-Zubereitung) der Firma Lukas Meyer, Magnafloc 1797 (Wässrige Lösung kationischer quervernetzter Kondensationsharze) der Firma Ciba Speciality Chemicals, Protectol KLC 50 (Dimethyl-C 12/14-alkylbenzylammoniumchlorid in Wasser (ca. 50%)) der Firma BASF, Rewoquat CPEM (Cocospentaethoxymethylammoniummethosulfat) der Firma Witco Surfactants GmbH, Rewoquat RTM 50 (Ricinolsäure propylamido trimethylammonium methosulfat) der Firma Witco Surfactants GmbH, Sochamine 35 (Alkylimidazolin) der Firma Witco Surfactants GmbH, eingesetzt werden.

Als nichtionisches Netzmittel kann eine Verbindung aus der Gruppe vernetzte Polyoxyethylenacrylsäure, Fettalkoholoxethylate, Nonylphenolpolyglycolether, Polyvinylpyrolidon, Glycerolfettsäureester, Propylenglykolfettsäureester, Sorbitanfettsäureester, Polyoxyethylensorbitanfettsäureester, Tetraoleinsäurepolyoxyethylensorbitol, Polyoxyethylenalkylether, Polyoxyethylenalkylphenylether, Polyoxyethylenpolyoxypropylenglykol, Polyoxyethylenpolyoxypropylenalkylether, Polyethylenglycolfettsäureester, höhere Fettsäurealkoholester, Polyhydricalkoholfettsäureester, eingesetzt werden.

Die Rußdispersion kann hergestellt werden, indem man den Ruß gemeinsam mit dem oder den Netzmitteln in Wasser dispergiert und gegebenenfalls für die Dispergierung Perlmühlen, Ultraschall-Geräte oder ein Ultra-Turrax verwendet. Im Anschluß an die Dispergierung kann die Rußdispersion zentrifugiert oder gefiltert werden.

Als primäres Amin der Formel R-NH₂ kann man alle Amine einsetzen, die Diazoniumsalze bilden können. Die Gruppe R kann eine aliphatische Gruppe und/oder cyklische, organische Gruppe sein. Die Gruppe R kann substituiert oder unsubstituiert, verzweigt oder unverzweigt sein. Aliphatische Gruppen können Alkane, Alkene, Alkohole, Ether, Aldehyde, Ketone, Karbonsäuren oder Kohlenhydrate sein.

Cyklische organische Gruppen können alicyklische Kohlenwasserstoffe, beispielsweise Cykloalkyle oder Cycloalkenyle, heterocyclische Kohlenwasserstoffe, beispielsweise Pyrrolidinyl, Pyrrolinyl, Piperidinyl oder Morpholinyl, Aryle, beispielsweise Phenyl, Naphtyl oder Anthracenyl oder Heteroaryle, beispielsweise Imidazolyl, Pyrazolyl, Pyridinyl, Thienyl, Thiazolyl, Furyl oder Indolyl, sein. Die Gruppe R kann substituiert sein mit R', OR',COR', COOR', OCOR', Carboxylat, Salze, beispielsweise COOLi, COONa, COOK oder COO⁻NR'₄⁺, OH, Halogen, CN, NR'₂, SO₃H, Sulfonatsalze, beispielsweise SO₃Li, SO₃Na, SO₃K, SO₃⁻ NR'₄⁺, OSO₃H oder OSO₃⁻Salze, NR'(COR'), CONR'₂, NO₂, PO₃H₂, Phosphonatsalze, beispielsweise PO₃HNa und PO₃Na₂, Phosphatsalze, beispielsweise OPO₃HNa und OPO₃Na₂, N=NR', NR'₃⁺X⁻, PR'₃⁺X⁻, SₖR', SSO₃H, SSO₃⁻Salze, SO₂NR'R'', SO₂SR', SNR'R'', SNQ, SO₂NQ, CO₂NQ, S-(1,4-piperazinediyl)-SR', 2-(1,3-dithianyl) 2-(1,3-dithiolanyl), SOR' und SO₂R'. R' und R" können gleich oder verschieden sein und H, unverzweigte oder verzweigte C₁-C₂₀ substituierte oder unsubstituierte, gesättigte oder ungesättigte Kohlenwasserstoffe, beispielsweise Alkyl, Alkenyl, Alkynyl, substituierte oder unsubstituierte Aryle, substituierte oder unsubstituierte Heteroaryle, substituierte oder unsubstituierte Alkylaryle oder substituierte oder unsubstituierte Arylalkyle sein und k eine ganze Zahl von 1-8, vorzugsweise 2-4, sein. Das Anion X⁻ kann ein Halogenid oder Anion einer mineral- oder organischen Säure sein. Q kann (CH₂)_{w}, (CH₂)ₓO(CH₂)_{z}, (CH₂)ₓNR(CH₂)_{z}, oder (CH₂)ₓS(CH₂)_{z}, wobei w eine Zahl von 2 bis 6 und x und z eine Zahl von 1 bis 6 ist, sein.

In einer Ausführungsform kann die Gruppe R eine aromatische Gruppe der Formel AyAr sein, wobei Ar ein aromatisches Radikal, beispielsweise Aryl oder Heteroaryl, vorzugsweise Phenyl, Naphthyl, Anthracenyl, Phenanthrenyl, Biphenyl, Pyridinyl, Benzothiadiazolyl oder Benzothiazolyl, A ein substituiertes aromatisches Radikal, wie zuvor beschrieben oder ein lineares, verzweigtes oder cyclisches Kohlenwasserstoffradikal, unsubstituiert oder substituiert mit ein oder mehreren funktionellen Gruppen und y eine ganze Zahl von 1 bis zur gesamten Zahl von -CH-Radikalen in dem aromatischen Radikal, ist.

In einer weiteren Ausführungsform kann die Gruppe R mit ionischen oder ionisierbaren Gruppen substituiert sein. Die ionische Gruppe kann eine anionische oder kationische Gruppe und die ionisierbare Gruppe ein Anion oder Kation sein, beispielsweise Sulfonsäure, Phosphorsäure, Carbonsäure, Sulfophenyl, vorzugsweise Hydroxysulfophenyl, substituiert oder unsubstituiertes Polysulfophenyl, substituiert oder unsubstituiertes Sulfonaphthyl, substituiert oder unsubstituiertes Polysulfonaphthyl oder deren Salze, p-Sulfophenyl (p-sulfanilsäure), 4-Hydroxy-3-sulfophenyl (2-hydroxy-5-amino-benzensulfonsäure), und 2-Sulfoethyl (2-aminoethansulfonsäure), quaternäre Ammoniumgruppe (-NR₃⁺) und quaternäre Phosphonium-Gruppen (-PR₃⁺), quaternäre zyklische Amine, N-substituiertes Pyridinverbindungen, beispielsweise N-methyl-pyridyl, (C₅H₄N) C₂H₅⁺, C₆H₄(NC₅H₅)⁺, C₆H₄COCH₂N(CH₃)₃⁺, C₆H₄COCH₂(NC₅H₅)⁺, (C₅H₄ N)CH₃⁺ und C₆H₄CH₂N(CH₃)₃⁺.

Die Gruppe R kann aromatische Sulfide, beispielsweise Ar(CH₂)_{q}Sₖ(CH₂)ᵣAr' oder A- (CH₂)_{q}S_{K}(CH₂)ᵣAr", wobei Ar und Ar' unabhängig voneinander substituiert oder unsubstituiert Arylen oder Heteroarylen Gruppen, Ar" eine Aryl oder Heteroaryl Gruppe ist, k ist 1 zu 8 und q und r sind 0-4, sein. Substituierte Arylgrupppen können substituierte Alkylaryl Gruppen enthalten, vorzugsweise bis-para-(C₆H₄)-S₂-(C₆H₄)-oder para (C₆H₄)-S₂-(C₆H₅) oder Aminophenyle, beispielsweise (C₆H₄)-NH₂, (C₆H₄)-CH₂-(C₆H₄)-NH₂ oder (C₆H₄)-SO₂-(C₆H₄)-NH₂.

In einer bevorzugten Ausführungsform kann als primäres Amin Verbindungen, die mindestens eine Hydroxi und mindestens eine Aminogruppe aufweist, beispielsweise Aminophenylethanol, oder Aminobenzolsulfonsäure verwendet werden.

Zur Einstellung des sauren pH-Wertes der wäßrigen Lösung oder Suspension des primären Amins können anorganische Säuren, vorzugsweise Salzsäure, verwendet werden. Der pH-Wert kann kleiner 6, vorzugsweise kleiner 3, sein.

Die Rußdispersion und die saure, wäßrige Lösung des primären Amins können unter Rühren gemischt werden.

Das Natriumnitrit kann in Wasser gelöst werden. Die Natriumnitrit-Lösung kann zur Rußdispersion/Amin-Mischung zugetropft werden. Die Umsetzung mit Natriumnitrit-Lösung kann bei Temperaturen von -5°C bis 30°C durchgeführt werden.

Die mit dem erfindungsgemäßen Verfahren hergestellte Rußdispersion kann je nach Anwendung direkt verwendet werden. Der modifizierte Ruß kann in einer Ausführungsform der Erfindung aus der Rußdispersion zurückgewonnen werden.

Der modifizierte Ruß kann sowohl als Füllstoff, Verstärkerfüllstoff, UV-Stabilisator, Leitfähigkeitsruß, als auch als Pigment in Kautschuk, Kunststoff, Druckfarben, Tinten, Inkjet-Tinten, Lacken, Farben, Bitumen, Beton, anderen Baustoffen und Papier eingesetzt werden.

Das erfindungsgemäße Verfahren weist den Vorteil auf, daß eine Umsetzung von Ruß auch mit Verbindungen, die mindestens eine Hydroxi und mindestens eine Aminogruppe aufweisen, möglich ist.

### Beispiele:

### Vergleichsbeispiel 1 gemäß WO 96/18688

100 g Ruß FW 18 werden im 3 l Becherglas in 1,4 l vollentsalztem Wasser suspendiert.
23,66g 2-(4-Aminophenyl)ethanol werden in 600 ml vollentsalztem Wasser aufgeschlämmt, 18 g Salzsäure (37 %) werden mit 10 ml vollentsalztem Wasser verdünnt und zur Amin-Suspension portionsweise zugegeben. Die Lösung wird klar und gelblich.

Zu dieser Lösung werden 10,4 g Natriumnitrit, gelöst in 104 ml vollentsalztem Wasser, bei Raumtemperatur zugetropft. Dabei verstärkt sich die Gelbfärbung, bis ein gelbes Substrat ausfällt. Durch Zugabe von verdünnter Salzsäure kann eine Wiederauflösung bewirkt werden. Dabei ist eine Bildung einer rötlichen, klebrigen Substanz zu beobachten. Diese Lösung wird zur Rußsuspension zugetropft. Eine Gasbildung kann beobachtet werden. Auf der Oberfläche scheidet sich eine rötliche, klebrige Substanz ab.

### Vergleichsbeispiel 2

100 g Ruß FW 18 werden in einem 3 l Becherglas in 1,4 l vollentsalztem Wasser suspendiert.
23,66 g 2-(4-Aminophenyl)ethanol werden mit 600 ml vollentsalztem Wasser aufgeschlämmt, 18 g Salzsäure (37 %) werden mit 10 ml vollentsalztem Wasser verdünnt und portionsweise zur Amin-Aufschlämmung zugegeben. Es entsteht eine gelbliche, klare Lösung, die unter Rühren zur Rußsuspension gegeben wird.

10,4 g Natriumnitrit werden in 104 ml vollentsalztem Wasser gelöst und in ca. 3 Stunden zum Gemisch aus Rußsupension und Aminophenylalkohol zugetropft. Dabei kann eine Blasenbildung beobachtet werden. Nach Rühren über Nacht (ca. 20 Stunden) wird die Suspension in zwei Abdampfschalen überführt und in einem Abluftrockenschrank bei 70 °C ca. 16 Stunden zur völligen Trocknung eingedampft.

Mit einem Labormixer wird der modifizierte Ruß bei Stufe III (Maximum) in zwei Portionen je 1 Minute aufgemahlen.

Eine Dispergierung und Stabilisierung des Rußes gelingt nicht einmal unter dem Einsatz von Netzmitteln, wie Hydropalat 3065 oder Tamol. Die einzelnen Rußteilchen sind durch gebildete "Polymere" derart miteinander fest verklebt, daß dieser Ruß im Gegensatz zu normalem Ruß nicht durch Ultraschall dispergiert werden kann.

### Vergleichsbeispiel 3

100 g Ruß FW 18 werden in einem 31 Becherglas in 1,4 l vollentsalztem Wasser suspendiert.
12 g 2-(4-Aminophenyl)ethanol werden mit 400 ml vollentsalztem Wasser aufgeschlämmt. 9 g Salzsäure (37 %) werden mit 20 ml vollentsalztem Wasser verdünnt und portionsweise zur Amin-Aufschlämmung zugegeben. Es entsteht eine klare, gelbliche Lösung, die unter Rühren zur Rußsuspension gegeben wird.
7,2 g Natriumnnitrit werden in 100 ml vollentsalztem Wasser gelöst und in ca. 2 Stunden zur Rußsuspension und Aminophenylalkohol zugetropft. Dabei kann eine Blasenbildung beobachtet werden. Nach Rühren über Nacht (ca. 20 Stunden) wird die Suspension in zwei Abdampfschalen überführt und in einem Ablufttrockenschrank bei 70°C ca. 16 Stunden zur völligen Trocknung eingedampft.

Mit einem Labormixer wird der modifizierte Ruß bei Stufe III (Maximum) in zwei Portionen je eine Minute aufgemahlen.

Gegenüber dem Vergleichsbeispiel 2 treten trotz der deutlich geringeren Konzentrationen an Aminophenylalkohol keinerlei Verbesserungen auf. Der Ruß ist nicht selbstdispergierend und lässt sich auch unter dem Einsatz von Netzmitteln nicht dispergieren.

### Beispiel 1

In die Lösung von 26,7 g Tamol in 540 g Wasser wird 100 g Ruß FW 18 bei laufendem Ultra Turrax (10 000 UPM) eingemischt und vordispergiert (30 min). Anschließend wird 2 mal mit Ultraschall im Durchfluss dispergiert. Unter dem Lichtmikroskop sind keine Teilchen > 1µ zu erkennen.

12 g 2-(4-Aminophenyl)ethanol werden in 400 ml vollentsalztem Wasser aufgeschlämmt. 9 g Salzsäure (37 %) werden mit 20 ml vollentsalztem Wasser verdünnt und portionsweise zur Amin-Aufschlämmung zugegeben. Es entsteht eine klare gelbliche Lösung, die unter Rühren zu der Rußdispersion gegeben wird.

7,24 g Natriumnitrit werden in 100 ml vollentsalztem Wasser gelöst und in ca. 1 Stunde zum Gemisch aus Rußdispersion und Aminohenylalkohol zugetropft. Man lässt über Nacht rühren.

### Beispiel 2

In das Gemisch von 66,6 g Hydropalat 3065 in 500 g Wasser wird 100 g Ruß FW 18 bei laufendem Ultra Turrax (10.000 UPM) eingemischt und vordispergiert (30 min). Anschließend wird 2 mal mit Ultraschall im Durchfluss dispergiert. Unter dem Lichtmikroskop sind keine Teilchen > 1 µ zu erkennen.

12 g 2-(4-Aminophenyl)ethanol werden in 400 ml vollentsalztem Wasser aufgeschlämmt, 9 g Salzsäure (37 %) werden mit 20 ml vollentsalztem Wasser verdünnt und protionsweise zur Amin-Aufschlämmung zugegeben. Es entsteht eine klare, gelbliche Lösung, die unter Rühren zur Rußdispersion gegeben wird.

7,24 g Natriumnitrit werden in 100 ml vollentsalztem Wasser gelöst und in ca. 2 Stunden zum Gemisch aus Rußdispersion und Aminophenylakohol zugetropft. Es tritt eine starke Schaumbildung auf. Man lässt über Nacht rühren.

Man erhält eine gefrierstabile Rußdispersion.

### Beispiel 3

In das Gemisch von 66,6 g Tego Dispers 750W in 500 g Wasser werden 100 g Ruß FW 18 bei laufendem Ultra Turrax (10.000 UPM) eingemischt und vordispergiert (30 min). Anschließend wird 2 mal mit Ultraschall im Durchfluss dispergiert. Es tritt eine starke Schaumbildung auf. Unter dem Lichtmikroskop sind keine Teilchen > 1µ zu erkennen.

20 g Sulfanilsäure werden in 700 ml vollentsalztem Wasser bei 65 °C gelöst. Nach dem Abkühlen bleibt die Lösung klar, obwohl es sich um eine übersättigte Lösung handelt. Diese Lösung wird unter Rühren zur Rußdispersion gegeben.

8,8 g Natriumnitrit werden in 100 ml vollentsalztem Wasser gelöst und in ca. 2 Stunden zum Gemisch aus Rußdispersion und Sulfanilsäure zugetropft. Es tritt eine starke Schaumbildung auf, die durch Zusatz von 1 ml UCO Foamaster 110.DE entschäumt wird. Man lässt 4 Tage nachrühren.

Man erhält eine gefrierstabile und lagerstabile Rußdispersion nach Zugabe von AMP 9.

Der Farbruß FW 18 ist ein Handelsprodukt der Firma Degussa.

Tamol ist ein anionisches Netzmittel und ist ein Natriumsalz eines Naphthalinsulfonsäureformaldehyd-Polykondensates. Hydropalat 3065 ist ein nicht ionisches Netzmittel und ist ein monofunktionelles Fettalkoholethoxylat-Blockcopolymer. Tego Dispers 750 W ist eine wäßrige Lösung eines organisch modifizierten Polymers mit pigmentaffinen Gruppen.

## Patentansprüche

1. Verfahren zur Herstellung von modifiziertem Ruß, **dadurch gekennzeichnet, daß** man eine Rußdispersion, enthaltend Ruß, Wasser und Netzmittel, mit einer sauren, wäßrigen Lösung oder Suspension eines primären Amins, das mindestens eine Hydroxi und mindestens eine Aminogruppe aufweist,mischt und anschließend mit Natriumnitrit-Lösung umsetzt.

2. Verfahren zur Herstellung von modifiziertem Ruß nach Anspruch 1, **dadurch gekennzeichnet, daß** man als Ruß Gasruß verwendet.

## Claims

1. Process for the production of modified black, **characterised in that** a black dispersion comprising black, water and wetting agent, is mixed with an acidic, aqueous solution or suspension of a primary amine having at least one hydroxy and at least one amino group and then reacted with sodium nitrite solution.

2. Process for the production of modified black according to claim 1, **characterised in that** gas black is used as the black.

## Revendications

1. Procédé de production de noir de carbone modifié,
**caractérisé en ce qu'**
on mélange une dispersion de noir de carbone contenant du noir de carbone, de l'eau et un agent de réticulation avec une solution aqueuse acide ou une suspension d'une amine primaire, qui présente au moins un hydroxy et au moins un groupe amino, et on la convertit ensuite à l'aide d'une solution de nitrite de sodium.

2. Procédé de production de noir de carbone modifié, selon la revendication 1,
**caractérisé en ce qu'**
on utilise comme noir de carbone du noir de fumée de gaz.
